# EUROPEAN PATENT SPECIFICATION

(11) **EP 3 943 599 B1**
(45) Date of publication and mention of the grant of the patent: **01.10.2025**
(21) Application number: 20774776.7
(22) Date of filing: 18.03.2020
(51) Int. Cl.: C12N 9/00, C07K 14/62, C12N 15/70

(54) **AMINOACYL-TRNA SYNTHETASE FOR EFFICIENTLY INTRODUCING LYSINE DERIVATIVE IN PROTEIN**
AMINOACYL-TRNA-SYNTHETASE ZUR EFFIZIENTEN EINFÜHRUNG VON LYSINDERIVAT IN PROTEIN
AMINOACYL-ARNT SYNTHÉTASE POUR L'INTRODUCTION EFFICACE D'UN DÉRIVÉ DE LYSINE DANS UNE PROTÉINE

(30) Priority: 19.03.2019 CN 201910209626
(43) Date of publication of application: 26.01.2022
(73) Proprietor: Ningbo Kunpeng Biotech Co., Ltd., Yuyao Ningbo, Zhejiang 315400 (CN)
(72) Inventor: WU, Song, Ningbo, Zhejiang 315400 (CN); ZHANG, Zhenshan, Ningbo, Zhejiang 315400 (CN); LIU, Huiling, Ningbo, Zhejiang 315400 (CN); CHEN, Wei, Ningbo, Zhejiang 315400 (CN)
(74) Representative: Lavoix
(86) International application number: PCT/CN2020/080034
(87) International publication number: WO 2020/187269

(56) References cited:
- EP-A1- 2 192 185
- WO-A1-2009/056803
- WO-A1-2015/193897
- WO-A1-2019/010164
- WO-A1-2019/010164
- WO-A2-2005/019415
- CN-A- 101 952 428
- CN-A- 104 099 360
- US-A1- 2011 136 168
- YANAGISAWA, T. ET AL.: "Multistep Engineering of Pyrrolysyl-tRNA Synthetase to Genetically Encode N3-(o-Azidobenzyloxycarbonyl) lysine for Site-Specific Protein Modification", CHEMISTRY & BIOLOGY, vol. 15, no. 11, 24 November 2008 (2008-11-24), pages 1191 - 1195, XP025678151, DOI: 10.1016/j.chembiol.2008.10.004
- YANAGISAWA, T. ET AL.: "Multistep Engineering of Pyrrolysyl-tRNA Synthetase to Genetically Encode N3-(o-Azidobenzyloxycarbonyl) lysine for Site-Specific Protein Modification", CHEMISTRY & BIOLOGY, vol. 15, no. 11, 24 November 2008 (2008-11-24), pages 1187 - 1197, XP025678151, DOI: 10.1016/j.chembiol.2008.10.004
- NEUMANN HEINZ; PEAK-CHEW SEW Y; CHIN JASON W: "Genetically Encoding Ne-acetyllysine in Recombinant Proteins", NATURE CHEMICAL BIOLOGY, vol. 4, no. 4, 17 February 2008 (2008-02-17) - 1 April 2008 (2008-04-01), pages 233 - 234, XP002518929
- NEUMANN, H. ET AL.: "Genetically Encoding Ne-acetyllysine in Recombinant Proteins", NATURE CHEMICAL BIOLOGY, vol. 4, no. 4, 17 February 2008 (2008-02-17), pages 232 - 234, XP002518929, DOI: 10.1038/NCHEMBIO.73

## Description

### Technical field

The present invention relates to the field of biotechnology, in particular to an aminoacyl-tRNA synthetase that efficiently introduces a lysine derivative into a protein.

### Background

The unnatural amino acid integrated protein (whole protein) obtained by replacing the amino acid residue at the desired position in the protein with the amino acids other than 20 types (unnatural amino acids) involved in normal protein synthesis can be used as an effective means for the analysis of protein structure and function. Using aminoacyl-tRNA synthetase (aaRS)/tRNA pairs derived from various biological species, more than 30 types of whole proteins have been synthesized. The system with the longest history and applied to the introduction of many useful unnatural amino acids is the tyrosyl-tRNA synthetase (TyrRS) mutant and amber suppressor gene tRNA^{Tyr} pair. As far as this method is concerned, the key is its orthogonal relationship, that is, aaRS in the two groups of eubacteria, archaea and eukaryotes aminoacylates tRNA in their respective groups, but the tRNAs of other groups cannot be aminoacylated.

On the other hand, the pyrrolysyl-tRNA synthetase (PylRS) and the amber suppressor gene tRNAPyl derived from Methanosarcina mazei function as an orthogonal aaRS/tRNA pair in E. coli cells. Pyrrolysine is a lysine derivative with a bulky methylpyrroline moiety on the side chain. Wild-type PylRS can bind Nε-Boc-L-lysine to tRNAPyl in E. coli. However, since LysRS has strict recognition of lysine, it has been difficult to introduce lysine derivatives having functional groups of various sizes and shapes into proteins site-specifically.

Therefore, the art needs to modify the wild-type lysyl-tRNA synthetase and develops an aminoacyl-tRNA synthetase that efficiently introduces lysine derivatives into the protein.

EP 2 192 185 A1 discloses a mutant pyrrolysyl-tRNA synthetase having a substitution of at least one amino acid residue selected from tyrosine at position 306, leucine at position 309 and cysteine at position 348 each constituting a pyrrolysine-binding site. This variant pyrrolysyl tRNA synthetase is obtained by modifying the wild-type pyrrolysyl tRNA synthetase derived from Methanosarcina genus.

US 2011/136168 A1 discloses a method of preparing non-natural protein including one or more ester bond in a polypeptide using a pyrrolysyl-tRNA synthetase derived from an archaebacterium, such as Methanosarcina mazei, or a mutant thereof comprising a substitution of at least one amino acid residue selected from alanine at position 302, tyrosine at position 306, leucine at position 309, asparagine at 346, cysteine at position 348, tyrosine at position 384 and tryptophan at 417, in the amino acid sequence of a wild type pyrrolysyl-tRNA synthetase of Methanosarcina mazei.

WO 2019/010164 A1 discloses a pyrrolysyl-tRNA synthetase (PylRS) variant having an amino acid substitution in one of the positions V31, T56, H62, or A100 and a chimeric PylRS variant which comprises a first portion comprising amino acid residues 1-149 of Methanosarcina barkeri Py1RS and a second portion comprising amino acid residues 185-454 of Methanosarcina mazei PylRS.

### Summary of the invention

The purpose of the present invention is to provide an aminoacyl-tRNA synthetase and a method for efficiently introducing lysine derivatives into proteins.

In a first aspect of the present invention, is provided a mutant lysyl-tRNA synthetase, the mutant lysyl-tRNA synthetase has a mutation at the position 19 arginine (R) and/or the position 29 histidine (H) in the amino acid sequence corresponding to the wild-type lysyl-tRNA synthetase.

The wild-type lysyl-tRNA synthetase is derived from Methanosarcina mazei.

The amino acid sequence of the wild-type lysyl-tRNA synthetase is shown in SEQ ID NO.: 1 or is shown in SEQ ID NO.: 2.

The arginine (R) at position 19 is mutated to histidine (H) or lysine (K); and/or
the histidine (H) at position 29 is mutated to arginine (R) or lysine (K).

The mutation in the mutant lysyl-tRNA synthetase is selected from the group consisting of R19H, R19K, H29R, H29K, and a combination thereof.

In a preferred embodiment, the mutant lysyl-tRNA synthetase further includes a mutation at a site selected from the group consisting of: isoleucine (I) at position 26, threonine (T) at position 122, Leucine (L) at position 309, Cysteine at position 348 (C), Tyrosine at position 384 (Y), and a combination thereof, wherein the isoleucine (I) at position 26 is mutated to valine (V), the threonine (T) at position 122 is mutated to tryptophan (S), leucine (L) at position 309 is mutated to Alanine (A), the cysteine (C) at position 348 is mutated to tryptophan (S), the tyrosine (Y) at position 384 is mutated to Phenylalanine (F).

In another preferred embodiment, the mutant lysyl-tRNA synthetase further includes a mutation selected from the group consisting of: 126V, T122S, L309A, C348S, Y384F, and a combination thereof.

In another preferred embodiment, the mutant lysyl-tRNA synthetase includes a mutation selected from the group consisting of R19H and H29R.

In another preferred embodiment, the mutant lysyl-tRNA synthetase includes a mutation selected from the group consisting of R19K and H29R.

In another preferred embodiment, the mutant lysyl-tRNA synthetase includes a mutation selected from the group consisting of R19H and H29K.

In another preferred embodiment, the mutant lysyl-tRNA synthetase includes a mutation selected from the group consisting of R19K and H29K.

In another preferred embodiment, the mutant lysyl-tRNA synthetase includes a mutation selected from the group consisting of R19H, I26V and H29R.

In another preferred embodiment, the mutant lysyl-tRNA synthetase includes a mutation selected from the group consisting of R19H, H29R, T122S and Y384F.

In another preferred embodiment, the mutant lysyl-tRNA synthetase includes a mutation selected from the group consisting of R19H, H29R, L309A and C348S.

The present disclosure further describes that, except for the mutation (such as, position 19 and/or 29, and optional position 26, 122, 309, 348 and/or 384), the other amino acids of the mutant lysyl-tRNA synthetase are substantially the same as the sequence as shown in SEQ ID NO.: 1 or SEQ ID NO.: 2.

In this instance, the substantially the same means that at most 50 (preferably 1-20, more preferably 1-10) amino acids are different, wherein the difference includes amino acid substitution, deletion or addition, and the mutant protein still has the activity of lysyl-tRNA synthetase.

In another instance, compared with SEQ ID NO. 1 or SEQ ID NO.: 2, the amino acid sequence of the mutant lysyl-tRNA synthetase has at least 70%, preferably at least 75%, 80%, 85%, 90%, and more preferably at least 95%, 96%, 97%, 98%, 99% sequence identity.

The mutant lysyl-tRNA synthetase is formed by mutation of the wild-type lysyl-tRNA synthetase as shown in SEQ ID NO.: 1 or SEQ ID NO.: 2.

In another preferred embodiment, the mutant lysyl-tRNA synthetase is selected from the group consisting of:
(1) a polypeptide having an amino acid sequence as shown in any one of SEQ ID NO.: 3-8; or
(2) a polypeptide formed by substituting, deleting or adding one or more (preferably 1-20, more preferably 1-15, more preferably 1-10, more preferably 1-8, more preferably 1-3, most preferably 1) amino acid residues to the amino acid sequence as shown in any one of SEQ ID NO.: 3-8, and derived from a polypeptide having the amino acid sequence as shown in any one of SEQ ID NO.: 3-8 having the function of the polypeptide as described in (1).

In another preferred embodiment, the amino acid sequence of the mutant lysyl-tRNA synthetase is shown in any one of SEQ ID NO.: 3-8.

In another preferred embodiment, the mutant lysyl-tRNA synthetase is an unnatural protein.

In another preferred embodiment, the mutant lysyl-tRNA synthetase is used to introduce lysine derivatives into the target protein.

In another preferred embodiment, the mutant lysyl-tRNA synthetase has the following characteristics:
compared with wild-type lysyl-tRNA synthetase, lysine derivatives with large functional groups can be introduced into proteins.

In a second aspect of the present invention, is provided an isolated polynucleotide encoding the mutant lysyl-tRNA synthetase according to the first aspect of the present invention.

In another preferred embodiment, the polynucleotide encodes a polypeptide as shown in any one of SEQ ID NO.: 3-8.

In another preferred embodiment, the polynucleotide includes a DNA sequence, an RNA sequence, and a combination thereof.

In another preferred embodiment, the polynucleotide additionally contains an auxiliary element selected from the group consisting of signal peptide, secretory peptide, tag sequence (such as 6His), and a combination thereof on the flanking of the ORF of the mutant lysyl-tRNA synthetase.

In a third aspect of the present invention, is provided a vector containing the polynucleotide according to the second aspect of the present invention.

In another preferred embodiment, the vector includes an expression vector, a shuttle vector, and an integration vector.

In another preferred embodiment, the vector is selected from the group consisting of pET, pCW, pUC, pPIC9k, pMA5, and a combination thereof.

In another preferred embodiment, the vector is pEvol vector and/or pBAD vector.

In another preferred embodiment, the vector is used to express mutant lysyl-tRNA synthetase.

In a fourth aspect of the present invention, is provided a host cell which contains the vector according to the third aspect of the present invention, or its genome integrates the polynucleotide according to the second aspect of the present invention.

In another preferred embodiment, the host cell is selected from the group consisting of a prokaryotic cell, a eukaryotic cell, and a combination thereof.

In another preferred embodiment, the host cell is a eukaryotic cell, such as a yeast cell, a plant cell, or an animal cell (such as a mammalian cell).

In another preferred embodiment, the host cell is a prokaryotic cell, such as Escherichia coli.

In another preferred embodiment, the host cell is E. coli Top10 or BL21.

In another preferred embodiment, the host cell comprises:
(a) the mutant lysyl-tRNA synthetase according to the first aspect of the present invention; and
(b) an orthogonal tRNA capable of binding to a lysine derivative in the presence of the mutant lysyl-tRNA synthetase; and optionally
(c) a target nucleic acid sequence encoding a target protein, which includes a codon recognized by the orthogonal tRNA at a position for introducing a lysine derivative.

In another preferred embodiment, the host cell further comprises:
(d) a lysine derivative.

In another preferred embodiment, the lysine derivative is a substrate of the mutant lysyl-tRNA synthetase.

The lysine derivative is selected from the group consisting of: alkynyloxycarbonyl lysine derivative, BOC-lysine (t-butyloxycarboryl -L-lysine) derivative, fatty acylated lysine, and a combination thereof.

In another preferred embodiment, the structure of the alkynyloxycarbonyl lysine is shown in Formula I: 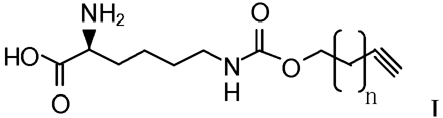 wherein n is 0-8.

In another preferred embodiment, the orthogonal tRNA is suppressor tRNA, preferably amber suppressor tRNA.

In another preferred embodiment, the encoding nucleic acid sequence of the orthogonal tRNA is shown in SEQ ID NO.: 9.

GGAAACCTGATCATGTAGATCGAATGGACTCTAAATCCGTTCAGCCGG
GTTAGATTCCCGGGGTTTCCGCCA (SEQ ID NO.: 9)

In another preferred embodiment, the codon recognized by the orthogonal tRNA is an amber codon.

In another preferred embodiment, the host cell is a prokaryotic cell or a eukaryotic cell, preferably Escherichia coli.

In a fifth aspect of the present invention, is provided an expression system, and the expression system comprises:
(a) the mutant lysyl-tRNA synthetase according to the first aspect of the present invention; and
(b) an orthogonal tRNA capable of binding to a lysine derivative in the presence of the mutant lysyl-tRNA synthetase; and optionally
(c) a target nucleic acid sequence encoding a target protein, which includes a codon recognized by the orthogonal tRNA at a position for introducing a lysine derivative.

In another preferred embodiment, the expression system further comprises:
(d) a lysine derivative.

The lysine derivative is selected from the group consisting of: alkynyloxycarbonyl lysine derivative, BOC-lysine (t-butyloxycarboryl -L-lysine) derivative, fatty acylated lysine, and a combination thereof.

In another preferred embodiment, the structure of the alkynyloxycarbonyl lysine is shown in Formula I: 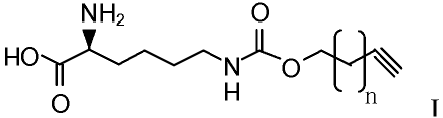 wherein n is 0-8.

In another preferred embodiment, the orthogonal tRNA is suppressor tRNA, preferably amber suppressor tRNA.

In another preferred embodiment, the nucleic acid sequence of the orthogonal tRNA is shown in SEQ ID NO.: 9.

In another preferred embodiment, the codon recognized by the orthogonal tRNA is an amber codon.

In another preferred embodiment, the expression system is a cell or a cell extract.

In another preferred embodiment, the expression system is used to introduce alkynyloxycarbonyl lysine into a target protein or prepare a target protein containing an unnatural amino acid.

The present disclosure further describes a method for introducing an unnatural amino acid into a target protein, including the steps:
(1) providing the host cell according to the fourth aspect of the present invention or the expression system according to the fifth aspect of the present invention; and
(2) in the presence of a lysine derivative, the target protein is expressed in the host cell or expression system.

In an instance, the target protein is selected from the group consisting of: human insulin precursor protein, insulin lispro precursor protein, insulin glargine precursor protein, parathyroid hormone, corticorelin, calcitonin, bivalirudin, glucagon-like peptide and its derivatives exenatide and liraglutide, ziconotide, sermorelin, somatorelin, secretin, teduglutide, Hirudin, growth hormone, growth factor, growth hormone releasing factor, adrenocorticotrophic hormone, release factor, deslorelin, desmopressin, elcatonin, glucagon, leuprorelin, luteinizing hormone-releasing hormone, somatostatyna, thyrotropin-releasing hormone, triptorelin, vasoactive intestinal peptide, interferon, parathyroid hormone, BH3 peptide, amyloid peptide, or a fragment of the above peptide, and a combination thereof.

In another instance, the unnatural amino acid is a lysine derivative.

In another instance, the method includes the steps:
(1) providing a cell or cell extract expressing the mutant lysyl-tRNA synthetase, orthogonal tRNA, and target nucleic acid sequence according to the first aspect of the present invention; and
(2) culturing the cell or cell extract in the presence of a lysine derivative, thereby introducing the lysine derivative into the target protein through the orthogonal tRNA synthetase and orthogonal tRNA pair.

In a sixth aspect of the present invention, is provided a kit comprising (a) a container, and (b) the mutant lysyl-tRNA synthetase according to the first aspect of the present invention or the polynucleotide according to the second aspect of the present invention located in the container.

In another preferred embodiment, the kit includes:
(i) a first nucleic acid sequence containing a first expression cassette for expressing the mutant lysyl-tRNA synthetase; and/or
(ii) a second nucleic acid sequence containing a second expression cassette for expressing an orthogonal tRNA, wherein the orthogonal tRNA can be combined with a lysine derivative in the presence of the mutant lysyl-tRNA synthetase; and/or
(iii) a third nucleic acid sequence, which contains a third expression cassette for expressing the target protein, and the coding sequence of the target protein includes a codon recognized by the orthogonal tRNA at the position for introducing the lysine derivative.

In another preferred embodiment, the kit further includes a cell extract.

In another preferred embodiment, the respective nucleic acid sequences are located in the same or different containers.

In another preferred embodiment, each nucleic acid sequence is located on a different vector.

The present disclosure further describes a translation system, and the translation system includes:
(a) the mutant lysyl-tRNA synthetase according to the first aspect of the present invention;
(b) an orthogonal tRNA capable of binding to a lysine derivative in the presence of the mutant lysyl-tRNA synthetase; and
(c) a lysine derivative.

In a seventh aspect of the present invention, is provided the use of the mutant lysyl-tRNA synthetase according to the first aspect of the present invention, or the host cell according to the fourth aspect of the present invention, or the expression system according to the fifth aspect of the present invention or the kit according to the sixth aspect of the present invention for incorporating an unnatural amino acid into a target protein or for preparing a target protein containing an unnatural amino acid.

The present disclosure further describes a method for preparing a target protein containing an unnatural amino acid, including the steps:
(1) providing the host cell according to the fourth aspect of the present invention or the expression system according to the fifth aspect of the present invention; and
(2) In the presence of a lysine derivative, the target protein is expressed in the host cell or expression system.

The present disclosure further describes a method for producing the mutant lysyl-tRNA synthetase according to the first aspect of the present invention, comprising the steps of: (i) culturing the host cell according to the fourth aspect of the present invention, thereby expressing the mutant lysyl-tRNA synthetase.

The present disclosure further describes an enzyme preparation containing the mutant lysyl-tRNA synthetase according to the first aspect of the present invention.

In an instance, the dosage form of the pharmaceutical preparation includes: a lyophilized preparation, a liquid preparation, and a combination thereof.

It should be understood that within the scope of the present invention, the above-mentioned technical features of the present invention and the technical features specifically described in the following (such as the embodiments) can be combined with each other to form a new or preferred technical solution. The invention is defined by the appended claims.

### Description of the drawings

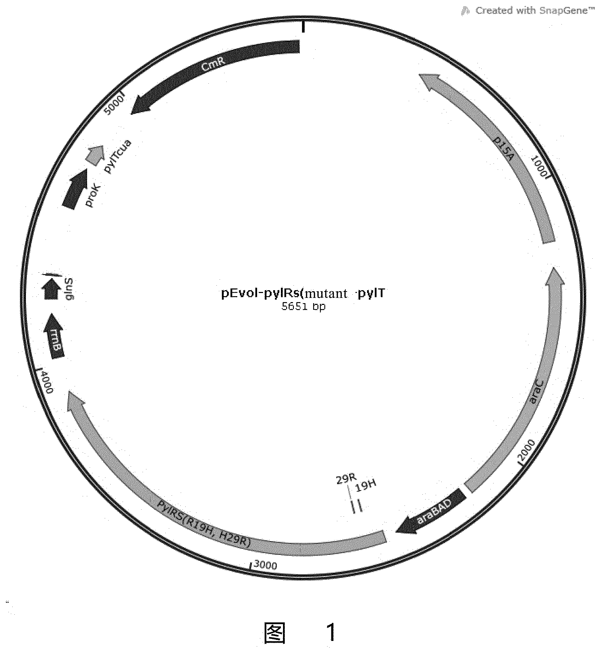

Figure 1 shows the map of plasmid pEvol-pylRs(R19H, H29R)-pylT.

### Detailed Description

After extensive and in-depth research and extensive screening, the inventors have unexpectedly obtained a mutant lysyl-tRNA synthetase. Compared with the wild-type lysyl-tRNA synthetase, the mutant lysyl-tRNA synthetase of the present invention can significantly increase the insertion amount of unnatural amino acids and the amount of target protein containing unnatural amino acids. In addition, the mutant lysyl-tRNA synthetase of the present invention can also improve the stability of the target protein so that it is not easily to break. On this basis, the inventors have completed the present invention.

### Terms

In order to make it easier to understand the present disclosure, first define certain terms. As used in this application, unless expressly stated otherwise herein, each of the following terms shall have the meaning given below. Other definitions are stated throughout the application.

The term "about" may refer to a value or composition within an acceptable error range of a particular value or composition determined by a person of ordinary skill in the art, which will depend in part on how the value or composition is measured or determined. For example, as used herein, the expression "about 100" includes all values between 99 and 101 (such as 99.1, 99.2, 99.3, 99.4, etc.).

As used herein, the term "containing" or "comprising (including)" can be open, semi-closed, and closed. In other words, the term also includes "substantially consisting of" or "consisting of".

Sequence identity (or homology) is determined by comparing two aligned sequences along a predetermined comparison window (it can be 50%, 60%, 70%, 80%, 90%, 95% or 100% of the length of the reference nucleotide sequence or protein) and determining the number of positions where the same residue appears. Usually, this is expressed as a percentage. The measurement of sequence identity of nucleotide sequences is a method well known to those skilled in the art.

In this article, "aminoacyl-tRNA synthetase" and "lysyl-tRNA synthetase" are used interchangeably.

### Wild-type lysyl-tRNA synthetase

As used herein, "wild-type lysyl-tRNA synthetase" refers to a naturally-occurring aminoacyl-tRNA synthetase that has not been artificially modified, and its nucleotides can be obtained through genetic engineering techniques, such as genome sequencing, polymerase chain reaction (PCR), etc., and the amino acid sequence can be deduced from the nucleotide sequence. The source of the wild-type lysyl-tRNA synthetase is Methanosarcina mazei.

In a preferred embodiment of the present invention, the amino acid sequence of the wild-type lysyl-tRNA synthetase is shown in SEQ ID NO.: 1.

MDKKPLNTLISATGLWMSRTGTIHKIKHHEVSRSKIYIEMACGDHLVVNNS
RSSRTARALRHHKYRKTCKRCRVSDEDLNKFLTKANEDQTSVKVKVVSAPTRT
KKAMPKSVARAPKPLENTEAAQAQPSGSKFSPAIPVSTQESVSVPASVSTSISSIS
TGATASALVKGNTNPITSMSAPVQASAPALTKSQTDRLEVLLNPKDEISLNSGKP
FRELESELLSRRKKDLQQIYAEERENYLGKLEREITRFFVDRGFLEIKSPILIPLEYI
ERMGIDNDTELSKQIFRVDKNFCLRPMLAPNLYNYLRKLDRALPDPIKIFEIGPC
YRKESDGKEHLEEFTMLNFCQMGSGCTRENLESIITDFLNHLGIDFKIVGDSCMV
YGDTLDVMHGDLELSSAVVGPIPLDREWGIDKPWIGAGFGLERLLKVKHDFKNI
KRAARSESYYNGISTNL (SEQ ID NO.: 1)

Alternatively, the amino acid sequence of the wild-type lysyl-tRNA synthetase is shown in SEQ ID NO.: 2.

MDKKPLNTLISATGLWMSRTGTIHKIKHHEVSRSKIYIEMACGDHLVVNNS
RSSRTARALRHHKYRKTCKRCRVSDEDLNKFLTKANEDQTSVKVKVVSAPTRT
KKAMPKSVARAPKPLENTEAAQAQPSGSKFSPAIPVSTQESVSVPASVSTSISSIS
TGATASALVKGNTNPITSMSAPVQASAPALTKSQTDRLEVLLNPKDEISLNSGKP
FRELESELLSRRKKDLQQIYAEERENYLGKLEREITRFFVDRGFLEIKSPILIPLEYI
ERMGIDNDTELSKQIFRVDKNFCLRPMLAPNLYNYLRKLDRALPDPIKIFEIGPC
YRKESDGKEHLEEFTMLNFCQMGSGCTRENLESIITDFLNHLGIDFKIVGDSCMV
YGDTLDVMHGDLELSSAVVGPIPLDREWGIDKPWIGAGFGLERLLKVKHDFKNI
KRAARSGSYYNGISTNL (SEQ ID NO.: 2)

### Mutant lysyl-tRNA synthetase

As used herein, the terms "mutant protein", "mutant protein of the present invention", "mutated aminoacyl-tRNA synthetase of the present invention", "mutant lysyl-tRNA synthetase", "mutant enzyme", " Mutants of aminoyl-tRNA synthase" can be used interchangeably, and both refer to non-naturally occurring mutant aminoacyl-tRNA synthetase, and the mutant aminoacyl-tRNA synthetase is a protein artificially modified of the polypeptide as shown in SEQ ID NO.: 1 or SEQ ID NO.: 2. Specifically, the mutant aminoacyl-tRNA synthetase is as described in the first aspect of the present invention.

It should be understood that the amino acid numbering in the mutant lysyl-tRNA synthetase of the present invention is based on the wild-type lysyl-tRNA synthetase ( SEQ ID NO.: 1 or SEQ ID NO.: 2). When a specific mutant protein has 80% or more homology with the sequence shown in SEQ ID NO.: 1 or SEQ ID NO.: 2, the amino acid number of the mutant protein may be misplaced relative to the amino acid numbering of SEQ ID NO.: 1 or SEQ ID NO.: 2., such as misalignment of positions 1-5 to the N-terminus or C-terminus of the amino acid, and using conventional sequence alignment techniques in the art, those skilled in the art can generally understand that such a misalignment is within a reasonable range and muteins with the same or similar glycosyltransferase activity with 80% homology should not be excluded from the scope of the mutant protein of the present invention due to the misplacement of amino acid numbering.

The mutant protein of the present invention is a synthetic protein or a recombinant protein, that is, it can be a chemically synthesized product, or produced from a prokaryotic or eukaryotic host (for example, bacteria, yeast, and plants) using recombinant technology. Depending on the host used in the recombinant production protocol, the mutein of the present invention may be glycosylated or non-glycosylated. The mutein of the present invention may also include or not include the initial methionine residue.

The present invention also includes fragments, derivatives and analogs of the mutein. As used herein, the terms "fragment", "derivative" and "analog" refer to a protein that substantially retains the same biological function or activity as the mutein.

The mutein fragment, derivative or analogue of the present invention may be (i) a mutein in which one or more conservative or non-conservative amino acid residues (preferably conservative amino acid residues) are substituted, and such substituted amino acid residues may or may not be encoded by the genetic condon, or (ii) a mutein with a substitution group in one or more amino acid residues, or (iii) a mutein formed by fusion of a mature mutein with another compound (such as a compound that prolongs the half-life of the mutein, such as polyethylene glycol), or (iv) the mutein formed by fusion of additional amino acid sequence to this mutein sequence (such as leader sequence or secretory sequence or sequence used to purify this mutant protein or proprotein sequence, or fusion protein formed with antigen IgG fragment). According to the teachings herein, these fragments, derivatives and analogs fall within the scope of those skilled in the art. In the present invention, conservatively substituted amino acids are preferably generated by amino acid substitutions according to Table I.

**Table I**

| Initial residues | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

The recognition of the amino acid substrate of PylRS is related to the stereochemical structure of the catalytically active functional domain. The size of lysine derivatives that can be activated by wild-type PylRS is limited, and lysine derivatives with large functional groups cannot be introduced into proteins. Therefore, by mutating the PylRS site, avoiding the steric hindrance of the binding substrate, or the interaction of the mutant amino acid with the substrate amino acid or the main chain part, to improve the effect.

Preferably, the mutein is as shown in any one of SEQ ID NO.: 3-8.

MDKKPLNTLISATGLWMSHTGTIHKVKHREVSRSKIYIEMACGDHLVVNN
SRSSRTARALRHHKYRKTCKRCRVSDEDLNKFLTKANEDQTSVKVKVVSAPTR
TKKAMPKSVARAPKPLENTEAAQAQPSGSKFSPAIPVSTQESVSVPASVSTSISSI
STGATASALVKGNTNPITSMSAPVQASAPALTKSQTDRLEVLLNPKDEISLNSGK
PFRELESELLSRRKKDLQQIYAEERENYLGKLEREITRFFVDRGFLEIKSPILIPLE
YIERMGIDNDTELSKQIFRVDKNFCLRPMLAPNLYNYLRKLDRALPDPIKIFEIGP
CYRKESDGKEHLEEFTMLNFCQMGSGCTRENLESIITDFLNHLGIDFKIVGDSCM
VYGDTLDVMHGDLELSSAVVGPIPLDREWGIDKPWIGAGFGLERLLKVKHDFK
NIKRAARSESYYNGISTNL (SEQ ID NO.: 3)

MDKKPLNTLISATGLWMSHTGTIHKIKHREVSRSKIYIEMACGDHLVVNNS
RSSRTARALRHHKYRKTCKRCRVSDEDLNKFLTKANEDQTSVKVKVVSAPTRT
KKAMPKSVARAPKPLENSEAAQAQPSGSKFSPAIPVSTQESVSVPASVSTSISSIS
TGATASALVKGNTNPITSMSAPVQASAPALTKSQTDRLEVLLNPKDEISLNSGKP
FRELESELLSRRKKDLQQIYAEERENYLGKLEREITRFFVDRGFLEIKSPILIPLEYI
ERMGIDNDTELSKQIFRVDKNFCLRPMLAPNLYNYLRKLDRALPDPIKIFEIGPC
YRKESDGKEHLEEFTMLNFCQMGSGCTRENLESIITDFLNHLGIDFKIVGDSCMV
FGDTLDVMHGDLELSSAVVGPIPLDREWGIDKPWIGAGFGLERLLKVKHDFKNI
KRAARSESYYNGISTNL (SEQ ID NO.: 4)

MDKKPLNTLISATGLWMSHTGTIHKIKHREVSRSKIYIEMACGDHLVVNNS
RSSRTARALRHHKYRKTCKRCRVSDEDLNKFLTKANEDQTSVKVKVVSAPTRT
KKAMPKSVARAPKPLENTEAAQAQPSGSKFSPAIPVSTQESVSVPASVSTSISSIS
TGATASALVKGNTNPITSMSAPVQASAPALTKSQTDRLEVLLNPKDEISLNSGKP
FRELESELLSRRKKDLQQIYAEERENYLGKLEREITRFFVDRGFLEIKSPILIPLEYI
ERMGIDNDTELSKQIFRVDKNFCLRPMLAPNLYNYARKLDRALPDPIKIFEIGPC
YRKESDGKEHLEEFTMLNFSQMGSGCTRENLESIITDFLNHLGIDFKIVGDSCMV
YGDTLDVMHGDLELSSAVVGPIPLDREWGIDKPWIGAGFGLERLLKVKHDFKNI
KRAARSESYYNGISTNL (SEQ ID NO.: 5)

MDKKPLNTLISATGLWMSHTGTIHKIKHREVSRSKIYIEMACGDHLVVNNS
RSSRTARALRHHKYRKTCKRCRVSDEDLNKFLTKANEDQTSVKVKVVSAPTRT
KKAMPKSVARAPKPLENTEAAQAQPSGSKFSPAIPVSTQESVSVPASVSTSISSIS
TGATASALVKGNTNPITSMSAPVQASAPALTKSQTDRLEVLLNPKDEISLNSGKP
FRELESELLSRRKKDLQQIYAEERENYLGKLEREITRFFVDRGFLEIKSPILIPLEYI
ERMGIDNDTELSKQIFRVDKNFCLRPMLAPNLYNYLRKLDRALPDPIKIFEIGPC
YRKESDGKEHLEEFTMLNFCQMGSGCTRENLESIITDFLNHLGIDFKIVGDSCMV

YGDTLDVMHGDLELSSAVVGPIPLDREWGIDKPWIGAGFGLERLLKVKHDFKNI
KRAARSESYYNGISTNL (SEQ ID NO.: 6)

MDKKPLNTLISATGLWMSHTGTIHKIKHHEVSRSKIYIEMACGDHLVVNN
SRSSRTARALRHHKYRKTCKRCRVSDEDLNKFLTKANEDQTSVKVKVVSAPTR
TKKAMPKSVARAPKPLENTEAAQAQPSGSKFSPAIPVSTQESVSVPASVSTSISSI
STGATASALVKGNTNPITSMSAPVQASAPALTKSQTDRLEVLLNPKDEISLNSGK
PFRELESELLSRRKKDLQQIYAEERENYLGKLEREITRFFVDRGFLEIKSPILIPLE
YIERMGIDNDTELSKQIFRVDKNFCLRPMLAPNLYNYLRKLDRALPDPIKIFEIGP
CYRKESDGKEHLEEFTMLNFCQMGSGCTRENLESIITDFLNHLGIDFKIVGDSCM
VYGDTLDVMHGDLELSSAVVGPIPLDREWGIDKPWIGAGFGLERLLKVKHDFK
NIKRAARSESYYNGISTNL (SEQ ID NO.: 7)

MDKKPLNTLISATGLWMSRTGTIHKIKHREVSRSKIYIEMACGDHLVVNNS
RSSRTARALRHHKYRKTCKRCRVSDEDLNKFLTKANEDQTSVKVKVVSAPTRT
KKAMPKSVARAPKPLENTEAAQAQPSGSKFSPAIPVSTQESVSVPASVSTSISSIS
TGATASALVKGNTNPITSMSAPVQASAPALTKSQTDRLEVLLNPKDEISLNSGKP
FRELESELLSRRKKDLQQIYAEERENYLGKLEREITRFFVDRGFLEIKSPILIPLEYI
ERMGIDNDTELSKQIFRVDKNFCLRPMLAPNLYNYLRKLDRALPDPIKIFEIGPC
YRKESDGKEHLEEFTMLNFCQMGSGCTRENLESIITDFLNHLGIDFKIVGDSCMV
YGDTLDVMHGDLELSSAVVGPIPLDREWGIDKPWIGAGFGLERLLKVKHDFKNI
KRAARSESYYNGISTNL (SEQ ID NO.: 8)

It should be understood that, compared with the sequence as shown in SEQ ID NO.: 1 or SEQ ID NO.: 2, the mutein of the present invention generally has higher homology (identity). Preferably, the homology between the mutant protein and the sequence as shown in SEQ ID NO.: 1 or SEQ ID NO.: 2 is at least 80%, preferably at least 85%-90%, and more preferably at least 95%, more preferably, at least 98%, and most preferably, at least 99%.

In addition, the mutant protein of the present invention can also be modified. Modified (usually not changing the primary structure) forms include: chemically derived forms of mutein in vivo or in vitro, such as acetylation or carboxylation. Modifications also include glycosylation, such as those produced by glycosylation modifications during the synthesis and processing of the mutant protein or during further processing steps. This modification can be accomplished by exposing the mutein to an enzyme that performs glycosylation (such as a mammalian glycosylase or deglycosylase). Modified forms also include sequences with phosphorylated amino acid residues (such as phosphotyrosine, phosphoserine, phosphothreonine). It also includes mutant proteins that have been modified to increase their resistance to proteolysis or optimize their solubility.

The term "polynucleotide encoding mutant lysyl-tRNA synthetase" includes the polynucleotide encoding the mutant lysyl-tRNA synthetase of the present invention.

The present disclosure further describes variants of the above polynucleotides, which encode fragments, analogs and derivatives of polypeptides or muteins having the same amino acid sequence as the present invention. These nucleotide variants include substitution variants, deletion variants and insertion variants. As is known in the art, an allelic variant is an alternative form of a polynucleotide. It may be a substitution, deletion or insertion of one or more nucleotides, but it will not substantially change the function of the mutant protein it encodes.

The present disclosure further describes polynucleotides that hybridize with the aforementioned sequences and have at least 50%, preferably at least 70%, and more preferably at least 80% identity between the two sequences. The present invention particularly relates to polynucleotides that can hybridize with the polynucleotide of the present invention under strict conditions (or stringent conditions). In the present invention, " strict conditions " refer to: (1) hybridization and elution at lower ionic strength and higher temperature, such as 0.2×SSC, 0.1% SDS, 60°C; or (2) adding denaturant during hybridization, such as 50% (v/v) formamide, 0.1% calf serum/0.1% Ficoll, 42°C, etc.; or (3) hybridization occurs only when the identity between the two sequences is at least 90% or more, and more preferably 95% or more.

The muteins and polynucleotides of the present invention are preferably provided in an isolated form, and more preferably, are purified to homogeneity.

The full-length sequence of the polynucleotide of the present invention can usually be obtained by PCR amplification method, recombination method or artificial synthesis method. For the PCR amplification method, primers can be designed according to the relevant nucleotide sequence disclosed in the present invention, especially the open reading frame sequence, and using a commercially available cDNA library or a cDNA library prepared according to a conventional method known to those skilled in the art as a template to amplify the relevant sequence. When the sequence is long, it is often necessary to perform two or more PCR amplifications, and then each amplified fragments are spliced together in the correct order.

Once the relevant sequence is obtained, the recombination method can be used to obtain the relevant sequence in large quantities. This is usually done by cloning it into a vector, then transferring it into a cell, and then isolating the relevant sequence from the proliferated host cell by conventional methods.

In addition, artificial synthesis methods can also be used to synthesize related sequences, especially when the fragment length is short. Usually, by first synthesizing multiple small fragments, and then ligating to obtain fragments with very long sequences.

At present, the DNA sequence encoding the protein (or fragment or derivative thereof) of the present invention can be obtained completely through chemical synthesis. The DNA sequence can then be introduced into various existing DNA molecules (or such as vectors) and cells known in the art. In addition, mutations can also be introduced into the protein sequence of the present invention through chemical synthesis.

The method of using PCR technology to amplify DNA/RNA is preferably used to obtain the polynucleotide of the present invention. Especially when it is difficult to obtain full-length cDNA from the library, the RACE method (RACE-cDNA end rapid amplification method) can be preferably used, and the primers used for PCR can be appropriately selected according to the sequence information of the present invention disclosed herein, and can be synthesized by conventional methods. The amplified DNA/RNA fragments can be separated and purified by conventional methods such as gel electrophoresis.

### Expression vector

The present invention also relates to a vector containing the polynucleotide of the present invention, a host cell produced by genetic engineering using the vector of the present invention or the mutant protein coding sequence of the present invention, and a method for producing the polypeptide of the present invention through recombinant technology.

Through conventional recombinant DNA technology, the polynucleotide sequence of the present invention can be used to express or produce recombinant mutein. Generally speaking, there are the following steps:
(1) using the polynucleotide (or variant) of the present invention encoding the mutant protein of the present invention, or using a recombinant expression vector containing the polynucleotide to transform or transduce a suitable host cell;
(2). a host cell cultured in a suitable medium;
(3). separating and purifying protein from culture medium or cells.

In the present invention, the polynucleotide sequence encoding the mutant protein can be inserted into a recombinant expression vector. The term "recombinant expression vector" refers to bacterial plasmids, bacteriophages, yeast plasmids, plant cell viruses, mammalian cell viruses such as adenovirus, retrovirus or other vectors well known in the art. Any plasmid and vector can be used as long as it can be replicated and stabilized in the host. An important feature of an expression vector is that it usually contains an origin of replication, a promoter, a marker gene, and translation control elements.

Methods well known to those skilled in the art can be used to construct an expression vector containing the DNA sequence encoding the mutein of the present invention and appropriate transcription/translation control signals. These methods include in vitro recombinant DNA technology, DNA synthesis technology, and in vivo recombination technology. The DNA sequence can be effectively linked to an appropriate promoter in the expression vector to guide mRNA synthesis. Representative examples of these promoters are: Escherichia coli lac or trp promoter; lambda phage PL promoter; eukaryotic promoters including CMV immediate early promoter, HSV thymidine kinase promoter, early and late SV40 promoter, retroviral LTRs and some other known promoters that can control gene expression in prokaryotic or eukaryotic cells or viruses. The expression vector also includes a ribosome binding site for translation initiation and a transcription terminator.

In addition, the expression vector preferably contains one or more selectable marker genes to provide phenotypic traits for selecting transformed host cells, such as dihydrofolate reductase for eukaryotic cell culture, neomycin resistance, and green fluorescent protein (GFP), or tetracycline or ampicillin resistance for E. coli.

A vector containing the above-mentioned appropriate DNA sequence and an appropriate promoter or control sequence can be used to transform an appropriate host cell so that it can express the protein.

The host cell can be a prokaryotic cell, such as a bacterial cell; or a lower eukaryotic cell, such as a yeast cell; or a higher eukaryotic cell, such as a mammalian cell. Representative examples include: Escherichia coli, Streptomyces; bacterial cells of Salmonella typhimurium; fungal cells such as yeast and plant cells (such as ginseng cells).

When the polynucleotide of the present invention is expressed in higher eukaryotic cells, if an enhancer sequence is inserted into the vector, the transcription will be enhanced. Enhancers are cis-acting factors of DNA, usually about 10 to 300 base pairs, acting on promoters to enhance gene transcription. Examples include the 100 to 270 base pairs of the SV40 enhancer on the late side of the replication initiation point, the polyoma enhancer on the late side of the replication initiation point, and adenovirus enhancers and the like.

Those of ordinary skill in the art know how to select appropriate vectors, promoters, enhancers and host cells.

Transformation of host cells with recombinant DNA can be carried out by conventional techniques well known to those skilled in the art. When the host is a prokaryote such as Escherichia coli, competent cells that can absorb DNA can be harvested after the exponential growth phase and treated with the CaCl₂ method. The steps used are well known in the art. Another method is to use MgCl₂. If necessary, transformation can also be carried out by electroporation. When the host is a eukaryote, the following DNA transfection methods can be selected: calcium phosphate co-precipitation method, conventional mechanical methods such as microinjection, electroporation, liposome packaging, etc.

The obtained transformants can be cultured by conventional methods to express the polypeptide encoded by the gene of the present invention. Depending on the host cell used, the medium used in the culture can be selected from various conventional mediums. The culture is carried out under conditions suitable for the growth of the host cell. After the host cells have grown to an appropriate cell density, the selected promoter is induced by a suitable method (such as temperature conversion or chemical induction), and the cells are cultured for a period of time.

The recombinant polypeptide in the above method can be expressed in the cell or on the cell membrane, or secreted out of the cell. If necessary, using its physical, chemical and other characteristics to separate and purify the recombinant protein through various separation methods. These methods are well known to those skilled in the art. Examples of these methods include, but are not limited to: conventional renaturation treatment, treatment with protein precipitation agent (salting out method), centrifugation, bacteria broken through osmosis, ultra-treatment, ultra-centrifugation, molecular sieve chromatography (gel filtration), adsorption chromatography, ion exchange chromatography, high performance liquid chromatography (HPLC) and various other liquid chromatography techniques and combinations of these methods.

Compared with the prior art, the present invention mainly has the following advantages:
After a large number of screenings and modifications, the present invention has first discovered that the mutation of arginine (R) at position 19 and/or histidine (H) at position 29 corresponding to wild-type lysyl-tRNA synthetase ( SEQ ID NO.: 1 or 2), the resulting mutant lysyl-tRNA synthetase can introduce lysine derivatives with large functional groups into proteins.

Compared with the wild-type lysyl-tRNA synthetase, the mutant lysyl-tRNA synthetase of the present invention can significantly increase the insertion amount of unnatural amino acids and the amount of target protein containing unnatural amino acids. In addition, the mutant lysyl-tRNA synthetase of the present invention can also improve the stability of the target protein so that it is not easily to break.

The present invention has also found that the mutation of one or more amino acids at other positions of lysyl-tRNA synthetase, such as positions 26, 122, 309, 348 and/or 384 alleles, can further increase the insertion amount of unnatural amino acids , the amount of target protein containing unnatural amino acids and/or the stability of the target protein.

The present invention will be further elaborated below in conjunction with specific embodiments. It should be understood that these embodiments are only used to illustrate the present invention and not to limit the scope of the present invention. The following examples do not specify the detailed conditions of the experimental methods, usually according to the conventional conditions such as the conditions described in the "Molecular Cloning Laboratory Guide" (translated by Huang Peitang et al., Beijing: Science Press, 2002) by Sambrook. J et al. , Or in accordance with the conditions recommended by the manufacturer (such as product specifications). Unless otherwise stated, percentages and parts are calculated by weight.

The experimental materials and reagents used in the following examples can be obtained from commercial channels unless otherwise specified.

### Example 1 Construction of mutants

According to the amino acid sequence 1 (SEQ ID NO.: 1) of the wild-type lysyl-tRNA synthetase pylRs, it was cloned into the SpeI-SalI site downstream of the araBAD promoter of the expression vector plasmid pEvol-pBpF (purchased from NTCC company, chloramphenicol resistance), wherein the SpeI restriction site was increased by PCR, and the SalI site was possessed by the vector itself. Keeping the original glutamine promoter glnS of the expression vector plasmid pEvol-pBpF. Downstream of the proK promoter of the expression vector plasmid pEvol-pBpF, the DNA sequence (SEQ ID NO.: 9) of the tRNA (pylTcua) of lysyl-tRNA synthetase was inserted by PCR. This plasmid was named pEvol-pylRs-pylT.

According to the amino acid sequence (SEQ ID NO.: 1) of the wild-type lysyl-tRNA synthetase pylRs, the mutation R19H was introduced to obtain the mutant lysyl-tRNA synthetase pylRs (R19H) with the amino acid sequence as shown in SEQ ID NO.: 7. According to the amino acid sequence (SEQ ID NO.: 1) of the wild-type lysyl-tRNA synthetase pylRs, the mutation H29R was introduced to obtain the mutant lysyl-tRNA synthetase pylRs (H29R) with the amino acid sequence as shown in SEQ ID NO.: 8. According to the amino acid sequence (SEQ ID NO.: 1) of wild-type lysyl-tRNA synthetase pylRs, mutations R19H, H29R were introduced, and the amino acid sequence of the mutant lysyl-tRNA synthetase pylRs (R19H, H29R) as shown in SEQ ID NO.: 6 was obtained. According to the amino acid sequence (SEQ ID NO.: 1) of wild-type lysyl-tRNA synthetase pylRs, mutations R19H, I26V, H29R were introduced to obtain the amino acid sequence of the mutant lysyl-tRNA synthetase pylRs (R19H, I26V, H29R) as shown in SEQ ID NO.: 3. According to the amino acid sequence (SEQ ID NO.: 1) of wild-type lysyl-tRNA synthetase pylRs, mutations R19H, H29R, T122S, Y384F were introduced, and the amino acid sequence of the mutant lysyl-tRNA synthetase pylRs (R19H, H29R, T122S, Y384F) as shown in SEQ ID NO.: 4 was obtained. According to the amino acid sequence (SEQ ID NO.: 1) of wild-type lysyl-tRNA synthetase pylRs, mutations R19H, H29R, L309A, C348S were introduced, and the amino acid sequence of the mutant lysyl-tRNA synthetase pylRs (R19H, H29R, L309A, C348S) as shown in SEQ ID NO.: 5 was obtained.

Using plasmid pEvol-pylRs-pylT as a template, designing primers, see SEQ ID NO.: 10 and SEQ ID NO.: 11, and introducing site-directed mutation R19H. The PCR products were treated with DpnI, separated by nucleic acid electrophoresis, extracted with agarose gel DNA recovery kit, ligated with T4 DNA Ligase, and transformed into large E.coli Top10 competent cells by chemical method (CaCl₂ method). The transformed cells were cultured on LB agar medium (10g/L yeast peptone, 5g/L yeast extract powder, 10g/L NaCl, 1.5% agar) containing chloramphenicol at 37°C overnight. A single live colony was picked and cultured in liquid LB medium (10g/L yeast peptone, 5g/L yeast extract powder, 10g/L NaCl) containing chloramphenicol at 37°C at 220 rpm overnight. The plasmid was extracted with a Plasmid Mini Extraction Kit, and the obtained plasmid was named pEvol-pylRs(R19H)-pylT;

Using plasmid pEvol-pylRs-pylT as a template, designing primers, see SEQ ID NO.: 12 and SEQ ID NO.: 13, introducing site-directed mutation H29R, and the resulting plasmid was named pEvol-pylRs(H29R)-pylT;

Using plasmid pEvol-pylRs(R19H)-pylT as a template, designing primers, see SEQ ID NO.: 12 and SEQ ID NO.: 13, introducing site-directed mutation H29R, and the resulting plasmid was named pEvol-pylRs (R19H, H29R)- pylT. The plasmid map is shown in Figure 1.
5'-ATACCGGCACCATCCACAAG-3' (SEQ ID NO.: 10)
5'-GGCTCATCCACAGACCAGTT -3' (SEQ ID NO.: 11)
5'-GCGAGGTTTCCCGTAGCAAA -3' (SEQ ID NO.: 12)
5'- GGTGTTTGATCTTGTGGATGGT-3' (SEQ ID NO.: 13)

Using the above methods to separately construct pEvol-pylRs (R19H, I26V, H29R)-pylT, plasmid pEvol-pylRs (R19H, H29R, T122S, Y384F)-pylT and plasmid pEvol-pylRs (R19H, H29R, L309A, C348S)-pylT.

### Example 2 Strain construction and high-density expression of tert-butoxycarbonyl (Boc) modified fusion protein

Plasmid pEvol-pylRs-pylT, plasmid pEvol-pylRs(R19H)-pylT, plasmid pEvol-pylRs (H29R)-pylT, plasmid pEvol-pylRs(R19H, H29R)-pylT, plasmid pEvol-pylRs(R19H, 126V, H29R)-pylT and plasmid pEvol-pylRs(R19H, H29R, T122S, Y384F)-pylT were respectively co-transformed with insulin fusion protein expression vector pBAD-INS (kanamycin resistance) into E.coli Top10 competent cells (competent cells purchased from Thermo) by chemical method (CaCl₂ method). The transformed cells were cultured on LB agar medium (10g/L yeast peptone, 5g/L yeast extract powder, 10g/L NaCl, 1.5% agar) containing 25µg/mL kanamycin and 17µg/mL chloramphenicol at 37° C overnight. Picking a single live colony and placing it in liquid LB medium (10g/L yeast peptone, 5g/L yeast extract powder, 10g/L NaCl) containing 25µg/mL kanamycin and 17µg/mL chloramphenicol at 37°C at 220rpm overnight. Adding 20% glycerol to the final concentration to preserve the strain.

Inoculating each strain in liquid LB medium at 37°C and 220 rpm overnight, inoculated at 1% (v/v) intank fermentation medium (12g/L yeast peptone, 24g/L yeast extract powder, 4mL/L glycerol, 12.8g/L disodium hydrogen phosphate, 3g/L potassium dihydrogen phosphate, 0.3 ‰ defoamer), cultured at 35(±3) °C, 200-1000 rpm, air flow 2-6 L/min. After culturing for 3-10 hours, the feed medium containing glycerol and yeast peptone was supplied at a stepped rate until the end of the fermentation. After culturing until OD₆₀₀ reaches 25-80, final concentration of 0.25% L-ara and final concentration of 5mM Boc-Lys were added for induction. Continued to cultivate until the OD₆₀₀ reaches 180-220, then put the tank. Then it was collected by centrifugation (5000rpm, 30min, 25°C). SDS-polyacrylamide electrophoresis was used to detect the expression of Boc-modified lysine-containing fusion proteins in whole cells of various strains.

The fusion protein is expressed in the form of insoluble "inclusion body". In order to release the inclusion body, the E. coli cells were disrupted with a high-pressure homogenizer. Nucleic acids, cell debris and soluble proteins are removed by centrifugation at 10000g. The inclusion bodies containing the fusion protein were washed with pure water, and the obtained inclusion body precipitate was used as the raw material for folding.

The expression levels of fusion proteins of different mutant enzymes are as follows:

| enzyme | Expression of Boc lysine fusion protein (g/L fermentation broth) |
|---|---|
| pylRs | 7.9 |
| R19H | 8.9 |
| H29R | 8.6 |
| R19H,H29R | 9.4 |
| R19H, I26V , H29R | 11.4 |
| R19H, H29R, T122S, Y384F | 14.0 |

In order to refold the fusion protein, the inclusion bodies were dissolved in a 7.5M urea solution pH 10.5 containing 2-10 mM mercaptoethanol so that the total protein concentration after dissolution was 10-25 mg/mL. The sample was diluted 5 to 10 times, and conventional folding was performed for 16 to 30 hours at 4 to 8°C andpH of 10.5 to 11.7. At 18-25°C, the pH value was maintained at 8.0-9.5, and using trypsin and carboxypeptidase B to hydrolyze the fusion protease for 10-20 hours, and then 0.45M ammonium sulfate was added to terminate the enzymatic hydrolysis reaction. Reversed-phase HPLC analysis results show that the yield of this enzymatic hydrolysis step is higher than 90%. The insulin analog obtained after digestion with trypsin and carboxypeptidase B is named BOC-lysine insulin. Boc-lysine insulin cannot be enzymatically hydrolyzed under the above conditions. The sample was clarified by membrane filtration, with 0.45 mM ammonium sulfate as a buffer, and initially purified by hydrophobic chromatography. The purity of SDS-polyacrylamide gel electrophoresis reaches 90% and the obtained Boc-human insulin was analyzed by MALDI-TOF mass spectrometry, and it is found that its molecular weight is consistent with the theoretical molecular weight of 5907.7 Da. The samples were collected by hydrophobic chromatography elution, and hydrochloric acid was added to carry out the Boc-human insulin deprotection reaction. Sodium hydroxide solution was added to control the pH to 2.8-3.2 to terminate the reaction. After two steps of high pressure reversed-phase chromatography, the yield of recombinant human insulin is higher than 85%.

The expression levels of recombinant human insulin for different mutant enzymes are as follows:

| enzyme | Human insulin production (mg/L fermentation broth) |
|---|---|
| pylRs | 360 |
| R19H | 440 |
| H29R | 400 |
| R19H, H29R | 450 |
| R19H, I26V, H29R | 580 |
| R19H, H29R, T122S, Y384F | 700 |

The results show that the use of the mutant enzyme of the present invention to prepare the target protein containing Boc-lysine can significantly increase the insertion amount of unnatural amino acids and the amount of target protein containing unnatural amino acids.

### Example 3 Strain construction and high-density expression of butynoxycarbonyl modified fusion protein

Plasmid pEvol-pylRs-pylT, plasmid pEvol-pylRs(R19H)-pylT, plasmid pEvol-pylRs(H29R)-pylT, plasmid pEvol-pylRs(R19H, H29R)-pylT, plasmid pEvol-pylRs(R19H29, 126V, H29R) )-pylT and plasmid pEvol-pylRs(R19H, H29R, L309A, C348S)-pylT, respectivelywere co-transformed with insulin fusion protein expression vector pBAD-INS (plasmid constructed by our company, kanamycin resistance) by chemical method (CaCl₂ method) into large E.coli Top10 competent cells (competent cells purchased from Thermo). The transformed cells were cultured on LB agar medium (10g/L yeast peptone, 5g/L yeast extract powder, 10g/L NaCl, 1.5% agar) containing 25µg/mL kanamycin and 17µg/mL chloramphenicol overnight at 37°C. Picking a single live colony and placing it in liquid LB medium (10g/L yeast peptone, 5g/L yeast extract powder, 10g/L NaCl) containing 25µg/mL kanamycin and 17µg/mL chloramphenicol overnight at 37°C, 220rpm. Adding 20% glycerol to the final concentration to preserve the strain.

Inoculating each strain in liquid LB medium at 37°C and 220 rpm overnight, inoculated at 1% (v/v) in tank fermentation medium (12g/L yeast peptone, 24g/L yeast extract powder, 4mL/L glycerol, 12.8g/L disodium hydrogen phosphate, 3g/L potassium dihydrogen phosphate, 0.3 ‰ defoamer), cultured at 35(±3) °C, 200-1000 rpm, air flow 2-6 L/min. After culturing for 3-10 hours, the feed medium containing glycerol and yeast peptone was supplied at a stepped rate until the end of the fermentation. After culturing until OD₆₀₀ reaches 25-80, final concentration of 0.25% L-ara and final concentration of 5mM Butynyloxycarbonyl-Lys were added for induction. Continued to cultivate until the OD₆₀₀ reaches 180-220, then put the tank. Then it was collected by centrifugation (5000rpm, 30min, 25°C). SDS-polyacrylamide electrophoresis was used to detect the expression of Butynyloxycarbonyl modified lysine fusion protein in whole cells of various strains.

| enzyme | Expression of butynoxycarbonyl lysine fusion protein (g/L fermentation broth) |
|---|---|
| pylRs | 4.5 |
| H29R | 5.0 |
| R19H | 5.2 |
| R19H, H29R | 6.8 |
| R19H,I26V , H29R | 6.7 |
| R19H, H29R, L309A, C348S | 8.4 |

The fusion protein is expressed in the form of insoluble "inclusion body". In order to release the inclusion body, the E. coli cells were disrupted with a high-pressure homogenizer. Nucleic acids, cell debris and soluble proteins are removed by centrifugation at 10000g. The inclusion bodies containing the fusion protein were washed with pure water, and the obtained inclusion body precipitate was used as the raw material for folding. In order to refold the fusion protein, dissolving the inclusion bodies in a 7.5M urea solution with pH 10.5 containing 2-10 mM mercaptoethanol so that the total protein concentration after dissolution was 10-25 mg/mL. The sample was diluted 5 to 10 times, and conventional folding was performed for 16 to 30 hours at 4 to 8°C and pH of 10.5 to 11.7. At 18-25°C, the pH value was maintained at 8.0-9.5, and using trypsin and carboxypeptidase B to hydrolyze the fusion protease for 10-20 hours, and then 0.45M ammonium sulfate was added to terminate the enzymatic hydrolysis reaction. Reversed-phase HPLC analysis results show that the yield of this enzymatic hydrolysis step is higher than 90%. The insulin analog obtained after digestion with trypsin and carboxypeptidase B is named Butynyloxycarbonyl-lysine insulin. Butynyloxycarbonyl-lysine insulin cannot be enzymatically hydrolyzed under the above conditions. The sample was clarified by membrane filtration, with 0.45 mM ammonium sulfate as a buffer, and initially purified by hydrophobic chromatography. The purity of SDS-polyacrylamide gel electrophoresis reaches 90% and the obtained Butynoxycarbonyl-human insulin was analyzed by MALDI-TOF mass spectrometry, and it is found that its molecular weight is consistent with the theoretical molecular weight of 5907.7 Da.

| enzyme | Production of butynoxycarbonyl human insulin (mg/L fermentation broth) |
|---|---|
| pylRs | 530 |
| H29R | 590 |
| R19H | 610 |
| R19H , H29R | 810 |
| R19H, I26V , H29R | 790 |
| R19H, H29R, L309A, C348S | 1000 |

The results show that the use of the mutant enzyme of the present invention to prepare butynoxycarbonyl modified target protein can significantly increase the insertion amount of unnatural amino acid and the amount of target protein containing unnatural amino acid.

## Claims

1. A mutant lysyl-tRNA synthetase, wherein the mutant lysyl-tRNA synthetase has a mutation at the position 19 arginine (R) and/or the position 29 histidine (H) in the amino acid sequence corresponding to the wild-type lysyl-tRNA synthetase,
wherein the amino acid sequence of the wild-type lysyl-tRNA synthetase is shown in SEQ ID NO.: 1 or 2,
wherein the arginine (R) at position 19 is mutated to histidine (H) or lysine (K); and/or
wherein the histidine (H) at position 29 is mutated to arginine (R) or lysine (K).

2. The mutant lysyl-tRNA synthetase of claim 1, wherein the mutant lysyl-tRNA synthetase further includes a mutation at a site selected from the group consisting of: isoleucine (I) at position 26, threonine (T) at position 122, leucine (L) at position 309, cysteine at position 348 (C), tyrosine at position 384 (Y), and a combination thereof;
wherein the isoleucine (I) at position 26 is mutated to valine (V), the threonine (T) at position 122 is mutated to tryptophan (S), leucine (L) at position 309 is mutated to alanine (A), the cysteine (C) at position 348 is mutated to tryptophan (S), the tyrosine (Y) at position 384 is mutated to phenylalanine (F).

3. The mutant lysyl-tRNA synthetase of claim 1, wherein the mutant lysyl-tRNA synthetase is
a polypeptide having an amino acid sequence as shown in any one of SEQ ID NO.: 3-8.

4. An isolated polynucleotide encoding the mutant lysyl-tRNA synthetase of claim 1.

5. A vector containing the polynucleotide of claim 4.

6. A host cell containing the vector of claim 5, or its genome integrates the polynucleotide of claim 4.

7. The host cell of claim 6, wherein the host cell comprises:
(a) the mutant lysyl-tRNA synthetase of claim 1; and
(b) an orthogonal tRNA capable of binding to a lysine derivative in the presence of the mutant lysyl-tRNA synthetase, wherein the lysine derivative is selected from the group consisting of: alkynyloxycarbonyl lysine, BOC-lysine (t-butyloxycarboryl -L-lysine), fatty acylated lysine, and a combination thereof; and optionally
(c) a target nucleic acid sequence encoding a target protein, which includes a codon recognized by the orthogonal tRNA at a position for introducing a lysine derivative.

8. An expression system comprising:
(a) the mutant lysyl-tRNA synthetase of claim 1; and
(b) an orthogonal tRNA capable of binding to a lysine derivative in the presence of the mutant lysyl-tRNA synthetase, wherein the lysine derivative is selected from the group consisting of: alkynyloxycarbonyl lysine, BOC-lysine (t-butyloxycarboryl -L-lysine), fatty acylated lysine, and a combination thereof.

9. The expression system of claim 8, further comprising:
(c) a target nucleic acid sequence encoding a target protein, which includes a codon recognized by the orthogonal tRNA at a position for introducing a lysine derivative.

10. A kit comprising (a) a container, and (b) the mutant lysyl-tRNA synthetase of claim 1 or the polynucleotide of claim 4 or the vector of claim 5 located in the container.

11. Use of the mutant lysyl-tRNA synthetase of claim 1, or the host cell of claim 6, or the expression system of claim 8 or 9 or the kit of claim 10 for incorporating an unnatural amino acid into a target protein or for preparing a target protein containing an unnatural amino acid.

## Patentansprüche

1. Mutierte Lysyl-tRNA-Synthetase, wobei die mutierte Lysyl-tRNA-Synthetase eine Mutation an der Position 19 Arginin (R) und/oder der Position 29 Histidin (H) in der Aminosäuresequenz aufweist, die der Wildtyp-Lysyl-tRNA-Synthetase entspricht,
wobei die Aminosäuresequenz der Wildtyp-Lysyl-tRNA-Synthetase gezeigt ist in SEQ ID NO.: 1 oder 2,
wobei das Arginin (R) an Position 19 zu Histidin (H) oder Lysin (K) mutiert ist; und/oder
wobei das Histidin (H) an Position 29 zu Arginin (R) oder Lysin (K) mutiert ist.

2. Mutierte Lysyl-tRNA-Synthetase nach Anspruch 1, wobei die mutierte Lysyl-tRNA-Synthetase ferner eine Mutation an einer Stelle beinhaltet, die ausgewählt aus der Gruppe, bestehend aus Isoleucin (I) an Position 26, Threonin (T) an Position 122, Leucin (L) an Position 309, Cystein an Position 348 (C), Tyrosin an Position 384 (Y) und einer Kombination davon, ist;
worin das Isoleucin (I) an Position 26 zu Valin (V) mutiert ist, das Threonin (T) an Position 122 zu Tryptophan (S) mutiert ist, Leucin (L) an Position 309 zu Alanin (A) mutiert ist, das Cystein (C) an Position 348 zu Tryptophan (S) mutiert ist, das Tyrosin (Y) an Position 384 zu Phenylalanin (F) mutiert ist.

3. Mutierte Lysyl-tRNA-Synthetase nach Anspruch 1, wobei die mutierte Lysyl-tRNA-Synthetase ein Polypeptid ist, das eine Aminosäuresequenz aufweist, wie sie in einer von SEQ ID NO.: 3-8 gezeigt ist.

4. Isoliertes Polynukleotid, das für die mutierte Lysyl-tRNA-Synthetase nach Anspruch 1 kodiert.

5. Vektor, der das Polynukleotid nach Anspruch 4 enthält.

6. Wirtszelle, die den Vektor nach Anspruch 5 enthält, oder deren Genom das Polynukleotid nach Anspruch 4 integriert.

7. Wirtszelle nach Anspruch 6, wobei die Wirtszelle Folgendes umfasst:
(a) die mutierte Lysyl-tRNA-Synthetase nach Anspruch 1; und
(b) eine orthogonale tRNA, die in der Lage ist, in Anwesenheit der mutierten Lysyl-tRNA-Synthetase an ein Lysinderivat zu binden, wobei das Lysinderivat ausgewählt ist aus der Gruppe, bestehend aus: Alkinyloxycarbonyl-Lysin, BOC-Lysin (t-Butyloxycarboryl-L-Lysin), fettacyliertem Lysin und einer Kombination davon; und optional
(c) eine Ziel-Nukleinsäuresequenz, die für ein Zielprotein kodiert, das ein Codon beinhaltet, das von der orthogonalen tRNA an einer Position zum Einführen eines Lysinderivats erkannt wird.

8. Expressionssystem, umfassend:
(a) die mutierte Lysyl-tRNA-Synthetase nach Anspruch 1; und
(b) eine orthogonale tRNA, die in der Lage ist, in Anwesenheit der mutierten Lysyl-tRNA-Synthetase an ein Lysin-Derivat zu binden, wobei das Lysin-Derivat ausgewählt ist aus der Gruppe, bestehend aus: Alkinyloxycarbonyl-Lysin, BOC-Lysin (t-Butyloxycarboryl-L-L-Lysin), fettacyliertem Lysin und einer Kombination davon.

9. Expressionssystem nach Anspruch 8, ferner umfassend:
(c) eine Ziel-Nukleinsäuresequenz, die für ein Zielprotein kodiert, das ein Codon beinhaltet, das von der orthogonalen tRNA an einer Position zum Einführen eines Lysinderivats erkannt wird.

10. Kit, umfassend (a) einen Behälter und (b) die mutierte Lysyl-tRNA-Synthetase nach Anspruch 1 oder das Polynukleotid nach Anspruch 4 oder den Vektor nach Anspruch 5, die sich in dem Behälter befinden.

11. Verwendung der mutierten Lysyl-tRNA-Synthetase nach Anspruch 1 oder der Wirtszelle nach Anspruch 6 oder des Expressionssystems nach Anspruch 8 oder 9 oder des Kits nach Anspruch 10 zum Integrieren einer unnatürlichen Aminosäure in ein Zielprotein oder zum Herstellen eines Zielproteins, das eine unnatürliche Aminosäure enthält.

## Revendications

1. Lysyl-ARNt synthétase mutante, dans laquelle la lysyl-ARNt synthétase mutante présente une mutation au niveau de l'arginine (R) en position 19 et/ou de l'histidine (H) en position 29 dans la séquence d'acides aminés correspondant à la lysyl-ARNt synthétase de type sauvage,
dans laquelle la séquence d'acides aminés de la lysyl-ARNt synthétase de type sauvage est représentée dans la SEQ ID NO : 1 ou 2,
dans laquelle l'arginine (R) en position 19 est mutée en histidine (H) ou en lysine (K) ; et/ou
dans laquelle l'histidine (H) en position 29 est mutée en arginine (R) ou en lysine (K).

2. Lysyl-ARNt synthétase mutante selon la revendication 1, dans laquelle la lysyl-ARNt synthétase mutante comprend en outre une mutation sur un site choisi dans le groupe constitué par : l'isoleucine (I) en position 26, la thréonine (T) en position 122, la leucine (L) en position 309, la cystéine en position 348 (C), la tyrosine en position 384 (Y), et une combinaison de celles-ci ;
dans laquelle l'isoleucine (I) en position 26 est mutée en valine (V), la thréonine (T) en position 122 est mutée en tryptophane (S), la leucine (L) en position 309 est mutée en alanine (A), la cystéine (C) en position 348 est mutée en tryptophane (S), la tyrosine (Y) en position 384 est mutée en phénylalanine (F).

3. Lysyl-ARNt synthétase mutante selon la revendication 1, dans laquelle la lysyl-ARNt synthétase mutante est un polypeptide ayant une séquence d'acides aminés telle qu'indiquée dans l'une quelconque des SEQ ID NO : 3 à 8.

4. Polynucléotide isolé codant pour la lysyl-ARNt synthétase mutante selon la revendication 1.

5. Vecteur contenant le polynucléotide selon la revendication 4.

6. Cellule hôte contenant le vecteur selon la revendication 5, ou dont le génome intègre le polynucléotide selon la revendication 4.

7. Cellule hôte selon la revendication 6, dans laquelle la cellule hôte comprend :
(a) la lysyl-ARNt synthétase mutante selon la revendication 1 ; et
(b) un ARNt orthogonal capable de se lier à un dérivé de lysine en présence de la lysyl-ARNt synthétase mutante, dans laquelle le dérivé de lysine est choisi dans le groupe constitué par : la alcynyloxycarbonyl-lysine, la BOC-lysine (t-butyloxycarbonyl -L-lysine), la lysine acylée grasse, et une combinaison de celles-ci ; et facultativement
(c) une séquence d'acide nucléique cible codant pour une protéine cible, qui comprend un codon reconnu par l'ARNt orthogonal à une position permettant d'introduire un dérivé de lysine.

8. Système d'expression comprenant :
(a) la lysyl-ARNt synthétase mutante selon la revendication 1 ; et
(b) un ARNt orthogonal capable de se lier à un dérivé de lysine en présence de la lysyl-ARNt synthétase mutante, dans lequel le dérivé de lysine est choisi dans le groupe constitué par : la alcynyloxycarbonyl-lysine, la BOC-lysine (t-butyloxycarbonyl -L-lysine), la lysine acylée grasse, et une combinaison de celles-ci.

9. Système d'expression selon la revendication 8, comprenant en outre :
(c) une séquence d'acide nucléique cible codant pour une protéine cible, qui comprend un codon reconnu par l'ARNt orthogonal à une position permettant d'introduire un dérivé de lysine.

10. Kit comprenant (a) un récipient, et (b) la lysyl-ARNt synthétase mutante selon la revendication 1 ou polynucléotide selon la revendication 4 ou vecteur selon la revendication 5 situé dans le récipient.

11. Utilisation de la lysyl-ARNt synthétase mutante selon la revendication 1, ou de la cellule hôte selon la revendication 6, ou du système d'expression selon la revendication 8 ou 9 ou du kit selon la revendication 10 pour incorporer un acide aminé non naturel dans une protéine cible ou pour préparer une protéine cible contenant un acide aminé non naturel.
